# EUROPEAN PATENT APPLICATION

(11) **EP 3 936 210 A1**
(43) Date of publication of application: **12.01.2022**
(21) Application number: 20205630.5
(22) Date of filing: 04.11.2020
(51) Int. Cl.: B01D 39/16, A41D 13/11, A62B 23/02

(54) **ANTIBACTERIAL DEODORIZING MASK INCLUDING TITANIUM DIOXIDE**

(30) Priority: 08.07.2020 KR 20200084394
(71) Applicant: Loesstech Co., Ltd., Daejeon (KR)
(72) Inventor: PARK, Ka Won, Daejeon (KR)
(74) Representative: Viering, Jentschura & Partner mbB Patent- und Rechtsanwälte

(57) **Abstract**

An antibacterial deodorizing mask including titanium dioxide according to the present disclosure includes: a mask body configured to surround at least one of a wearer's mouth and nose; and a pair of ear loops provided at both sides of the mask body so as to be hung on the wearer's ears and each having both ends coupled to upper and lower portions of the mask body, respectively, in which the mask body includes titanium dioxide particles having an average particle diameter D50 of 0.2 mm to 2 mm.

## Description

### CROSS-REFERENCE TO RELATED APPLICATIONS

This application claims the priority of Korean Patent Application No. 10-2020-0084394 filed on July 8, 2020, in the Korean Intellectual Property Office, the disclosure of which is incorporated herein by reference.

### BACKGROUND

### Field

The present disclosure relates to an antibacterial deodorizing mask including titanium dioxide.

### Description of the Related Art

Recently, there is rapidly increasing use of masks as fashion items as well as masks for avoiding coronaviruses, fine dust, yellow dust, environmental pollutants, and the like. Most of the masks are manufactured for single use, but in practice, changing masks every day causes a cost burden.

Recently, it is recommended to wear the mask anytime, anywhere, and every day, but a disposable mask is often used multiple times in a situation in which it is difficult to obtain the mask or because of the cost burden related to the mask.

The mask provides an environment in which bacteria and the like are suitable for breeding because of humidity from the wearer's exhalation and of exposure to food on a side of a mouth or in the mouth. Further, oral diseases, gastrointestinal diseases, and halitosis caused by food are transferred to the mask, and as a result, using a disposable mask multiple times rather harms health or causes discomfort.

Therefore, there is a need for an antibacterial deodorizing mask which is manufactured by providing antibacterial and deodorizing functions to the mask and may prevent bacterial growth and offensive odor and extend the lifespan of the mask even when the mask is worn over a long period of time.

Meanwhile, titanium dioxide is used as inorganic photocatalysts in various fields because of an excellent antibacterial and deodorizing effect thereof. However, recently, some studies are of opinion that titanium dioxide may degrade the immune system. These opinions and studies have not yet been verified, but it is reluctant to use titanium dioxide as it is, which is currently used in nanoparticle units.

Accordingly, there is also a need for a technology for applying titanium dioxide so that there is no likelihood that the human body inhales titanium dioxide.

### SUMMARY

An object to be achieved by the present disclosure is to provide an antibacterial deodorizing mask including titanium dioxide having an excellent antibacterial and deodorizing effect.

Another object to be achieved by the present disclosure is to provide an antibacterial deodorizing mask including titanium dioxide having a prolonged lifespan.

Still another object to be achieved by the present disclosure is to provide an antibacterial deodorizing mask including titanium dioxide, in which there is no likelihood that a wearer inhales titanium dioxide.

The above-mentioned objects and other objects of the present disclosure all may be achieved by the present disclosure to be described below.

One aspect of the present disclosure relates to an antibacterial deodorizing mask including titanium dioxide.

According to an exemplary embodiment, the antibacterial deodorizing mask including titanium dioxide includes: a mask body configured to surround at least one of a wearer's mouth and nose; and a pair of ear loops provided at both sides of the mask body so as to be hung on the wearer's ears and each having both ends coupled to upper and lower portions of the mask body, respectively, in which the mask body includes an antibacterial deodorizing layer including titanium dioxide particles having an average particle diameter D50 of 0.2 mm to 2 mm.

The mask body may include: an inner fabric; a filter formed on the inner fabric; and an outer fabric formed on the filter, and the antibacterial deodorizing layer may be formed on the filter so as to be directed toward the outer fabric or formed on the outer fabric so as to be directed toward the filter.

The filter and the outer fabric may be at least partially coupled by ultrasonic bonding.

The mask body may include: an inner fabric; a filter formed on the inner fabric; an intermediate material formed on the filter; and an outer fabric formed on the intermediate material, and the antibacterial deodorizing layer may be formed on the intermediate material so as to be directed toward the outer fabric.

The intermediate material and the outer fabric may be at least partially coupled by ultrasonic bonding.

Loess, charcoal, activated carbon, terra alba, zeolite, and lime particles may be attached to surfaces of the titanium dioxide particles by means of at least one of bentonite and montmorillonite.

A ratio of an average particle diameter D50 of the loess, the charcoal, the activated carbon, the terra alba, the zeolite, and the lime particles to an average particle diameter D50 of the titanium dioxide may be 0.1:1 to 0.3:1.

The titanium dioxide particles having the average particle diameter D50 of 0.2 mm to 2 mm may be made of a composition for forming titanium dioxide, and the composition for forming the titanium dioxide may include 85 to 95% by weight of titanium dioxide particles having an average particle diameter D50 of 1 µm or less and 5 to 15% by weight of magnesium.

The titanium dioxide particles may be manufactured by a method including: mixing 85 to 95% by weight of titanium dioxide particles having an average particle diameter D50 of 1 µm or less and 5 to 15% by weight of magnesium; heating the mixture in a tube furnace under a H₂/Ar atmosphere at 500°C to 550°C for 4 to 6 hours; stirring the mixture in a 1.0 M hydrogen chloride (HCl) solution for 11 to 13 hours; removing acid by washing the mixture with water; and drying the mixture.

Another aspect of the present disclosure relates to an antibacterial deodorizing pad.

According to an exemplary embodiment, the antibacterial deodorizing pad may include an intermediate material layer including spun bond, and an antibacterial deodorizing layer provided on the intermediate material layer and including titanium dioxide particles having an average particle diameter D50 of 0.2 mm to 2 mm.

The present disclosure advantageously provides the antibacterial deodorizing mask including titanium dioxide having an excellent antibacterial and deodorizing effect, having a prolonged lifespan, and making it impossible for a wearer to inhale titanium dioxide particles.

### BRIEF DESCRIPTION OF THE DRAWINGS

The above and other aspects, features and other advantages of the present disclosure will be more clearly understood from the following detailed description taken in conjunction with the accompanying drawings, in which:
FIG. 1 is a view simply illustrating an antibacterial deodorizing mask including titanium dioxide according to an exemplary embodiment of the present disclosure;
FIG. 2 is a view illustrating an exemplary embodiment of a cross section of a mask body taken along line S-S' in FIG. 1;
FIG. 3 is a view illustrating another exemplary embodiment of a cross section of the mask body taken along line S-S' in FIG. 1;
FIG. 4 is a view simply illustrating a titanium dioxide particle according to another exemplary embodiment of the present disclosure; and
FIG. 5 is a cross-sectional view simply illustrating an antibacterial deodorizing pad according to an exemplary embodiment of the present disclosure.

### DETAILED DESCRIPTION OF THE EMBODIMENT

Hereinafter, exemplary embodiments of the present application will be described in more detail with reference to the accompanying drawings. However, technologies disclosed in the present application are not limited to the exemplary embodiments described herein and may be specified as other aspects.

The exemplary embodiments introduced herein are merely provided to make the disclosed content thorough and complete, and sufficiently transfer the spirit of the present application those skilled in the art. In order to clearly illustrate constituent elements of each device in the drawings, a size, such as a width or a thickness, of the constituent element is somewhat enlarged. In addition, although only some of the constituent elements are illustrated for convenience of description, those skilled in the art will easily recognize the remaining constituent elements.

When the description is made as a whole from the observer's point of view when describing the drawings and when one element is described as being positioned above or below another element, this means both that one element is positioned directly above or below another element and that an additional element may be interposed between these elements. In addition, those skilled in the art may implement the spirit of the present application in various other forms without departing from the technical spirit of the present application. Further, the same reference numerals in the plurality of drawings refer to substantially the same elements.

Meanwhile, it should be understood that singular expressions disclosed in the present application include plural expressions unless clearly described as different meanings in the context. It should be understood that the terms "comprises, " "comprising," "includes," "including," "containing," "has," "having" or other variations thereof are inclusive and therefore specify the presence of stated features, integers, steps, operations, elements, components, and/or combinations thereof, but do not preclude the presence or addition of one or more other features, integers, steps, operations, elements, components, and/or combinations thereof.

In addition, in the present specification, the term 'X to Y', which represents a range, means 'X or more and Y or less' .

Throughout the present specification, when one constituent element is referred to as being "connected to" another constituent element, one constituent element can be "directly connected to" the other constituent element, and one constituent element can also be "electrically connected to" the other element with other elements therebetween. In addition, unless explicitly described to the contrary, the word "comprise/include" and variations such as "comprises/includes" or "comprising/including" will be understood to imply the inclusion of stated elements, not the exclusion of any other elements.

### ANTIBACTERIAL DEODORIZING MASK INCLUDING TITANIUM DIOXIDE

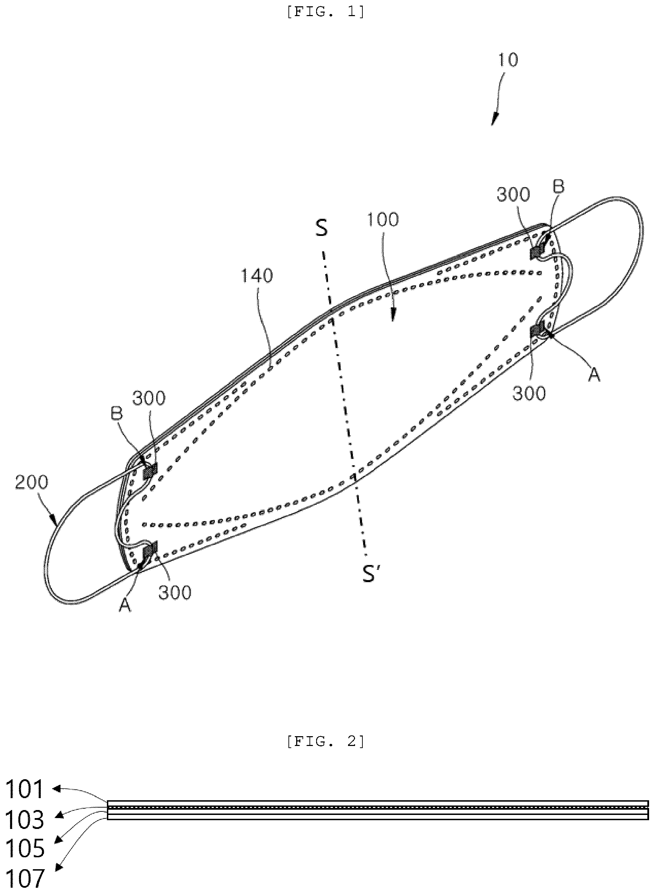

An antibacterial deodorizing mask including titanium dioxide according to an exemplary embodiment of the present disclosure will be described with reference to FIG. 1. FIG. 1 is a view simply illustrating an antibacterial deodorizing mask including titanium dioxide according to an exemplary embodiment of the present disclosure.

According to an exemplary embodiment of the present disclosure, an antibacterial deodorizing mask 10 including titanium dioxide includes a mask body 100 configured to surround at least one of a wearer's mouth and nose, and a pair of ear loops 200 provided at both sides of the mask body so as to be hung on the wearer's ears and each having both ends coupled to upper and lower portions of the mask body, respectively, in which the mask body 100 includes an antibacterial deodorizing layer including titanium dioxide particles having an average particle diameter D50 of 0.2 mm to 2 mm.

The mask body 100 surrounds at least one of the wearer's mouth and nose and serves to block viruses, fine dust, yellow dust, respiratory droplets, and the like. The mask body 100 according to the present disclosure includes the antibacterial deodorizing layer including the titanium dioxide particles having an average particle diameter D50 of 0.2 mm to 2 mm.

The titanium dioxide particles are inorganic photocatalysts and have an excellent antibacterial and deodorizing effect. However, the titanium dioxide particles have been used as nano-sized particles until now, and as a result, there is a great concern that the wearer may inhale the titanium dioxide particles when the titanium dioxide particles are applied to the mask. The titanium dioxide is known as a very stable and biologically harmless substance enough to be used as a food additive, but many people concern about the likelihood of inhalation of titanium dioxide because of the recent research results showing that the titanium dioxide may degrade the immune system.

The antibacterial deodorizing mask including titanium dioxide according to the present disclosure adopts the titanium dioxide particles having an average particle diameter D50 of 0.2 mm to 2 mm and thus has an advantage in that there is no likelihood that the wearer inhales the titanium dioxide particles. The average particle diameter D50 of the titanium dioxide particles may be 0.2 mm to 2 mm, particularly, 0.5 mm to 1.5 mm, and more particularly, 0.7 mm to 1.2 mm. In these ranges of the particle diameter, the antibacterial deodorizing effect of the titanium dioxide particles may be sufficiently exhibited, there is no concern that the wearer inhales the titanium dioxide particles, and it is possible to minimize a situation in which the titanium dioxide particles are collected at a lower side of the mask by gravity during the use of the mask.

A method of manufacturing the titanium dioxide particles having the average particle diameter D50 of 0.2 mm to 2 mm will be described below.

The ear loops 200 are provided to secure the mask body 100 on the wearer's face and configured to be hung on the wearer's ears. The ear loops 200 are provided at both sides in a horizontal direction of the mask body 100 and may be connected to the mask body 100.

Specifically, the ear loop 200 according to the present disclosure is described as having a closed curve shape, for example, a ring shape. In addition, the ear loop 200 may be made of an elastic material. When the ear loop 200 is pulled in a longitudinal direction thereof, a length of the ear loop 200 may be greater than a length in a state before the ear loop 200 is pulled (hereinafter, referred to as a 'basic state').

In this case, a thickness of the ear loop 200 may be decreased as the length of the ear loop 200 is increased. That is, the ear loop 200 is made of an elastic material of which a thickness is decreased and a length is increased when the ear loop 200 is pulled in comparison with the basic state.

The ear loops 200 may be connected to the mask body 100 by ear loop coupling parts 300 (A and B). That is, the ear loop coupling parts may be connected to the ear loops 200 and may connect the ear loops 200 to the mask body 100.

The number of ear loop coupling parts corresponds to the number of ear loops 200. In the present exemplary embodiment, the pair of ear loops 200 may be provided, one for each of the two opposite sides in the horizontal direction of the mask body 100, and the ear loop coupling parts may also be provided, one pair for each of the two opposite sides in the horizontal direction of the mask body 100 (A and B).

The ear loop coupling part 300 may be coupled to the mask body 100 such that a passage through which the ear loop extends has a width smaller than a thickness of the ear loop 200 in the basic state (e.g., a ratio of cross-sectional area is 1:0.8 to 1:0.9). That is, the ear loop coupling part 300 may be coupled to the mask body 100 such that a width of a space formed between the mask body 100 and the ear loop coupling part 300 is smaller than a thickness of the ear loop 200 in the basic state, that is, the state in which the ear loop 200 is not pulled in the longitudinal direction thereof.

Hereinafter, the configuration of the mask body 100 according to the exemplary embodiment the present disclosure will be described in more detail with reference to FIG. 2. FIG. 2 is a view illustrating an exemplary embodiment of a cross section of the mask body taken along line S-S' in FIG. 1.

Referring to FIG. 2, the mask body 100 according to the exemplary embodiment may include an inner fabric 107, a filter 105 formed on the inner fabric 107, and an outer fabric 101 formed on the filter 105, and the antibacterial deodorizing layer 103 may be formed on the filter 105 so as to be directed toward the outer fabric 101 or formed on the outer fabric 101 so as to be directed toward the filter 105.

The inner fabric 107 and the outer fabric 101 may adopt spun bond as a non-woven fabric, and for example, spun bond of 20 g to 40 g may be applied.

The filter 105 may be applied without limitation as long as the filter 105 may block at least one of fine dust, yellow dust, and respiratory droplets, and examples of the filter 105 may include at least one of an MB filter of 30 gsm and an MB filter of 40 gsm.

The antibacterial deodorizing layer 103 refers to a layer in which the titanium dioxide particles are dispersed. The titanium dioxide particles may be formed on the filter 105 so as to be directed toward the outer fabric 101 or formed on the outer fabric 101 so as to be directed toward the filter 105. Because fine fibers are entangled on a surface of the filter 105 and a surface of the outer fabric 101, the titanium dioxide particles having the average particle diameter D50 of 0.2 mm to 2 mm may be fixed without a separate medium. Specifically, plastic clay may be applied as a medium in order to improve fixing force, and for example, at least one of bentonite and montmorillonite may be applied.

Alternatively, in order to fix the titanium dioxide, the titanium dioxide particles are dispersed and applied onto the filter 105 and/or the outer fabric 101, and then air or gas is sprayed by an air gun or a gas gun of 0.1 J to 0.5 J, thereby increasing fixing force.

Alternatively, the titanium dioxide particles are sprayed onto the filter 105 and/or the outer fabric 101 by an air sand gun or an air spray gun to form the antibacterial deodorizing layer 103, thereby improving fixing force of the titanium dioxide particles.

In the exemplary embodiment, the filter 105 or the outer fabric 101 on which the titanium dioxide particles are formed may be manufactured separately in advance and then applied to a process of manufacturing the mask, and in this case, process characteristics may be further improved.

In addition, the filter 105 and the outer fabric 101 may be at least partially coupled by ultrasonic bonding 140. In this case, it is possible to further improve fixing force of the titanium dioxide particles (antibacterial deodorizing layer) included between the filter 105 and the outer fabric 101. FIG. 1 illustrates that the ultrasonic bonding 140 is performed only partially, but the ultrasonic bonding may be performed in the form of a lattice in order to improve fixing force of the titanium dioxide particles. For example, the ultrasonic bonding may be performed in the form of a lattice in which a length of one side is 8 mm to 12 mm (not illustrated).

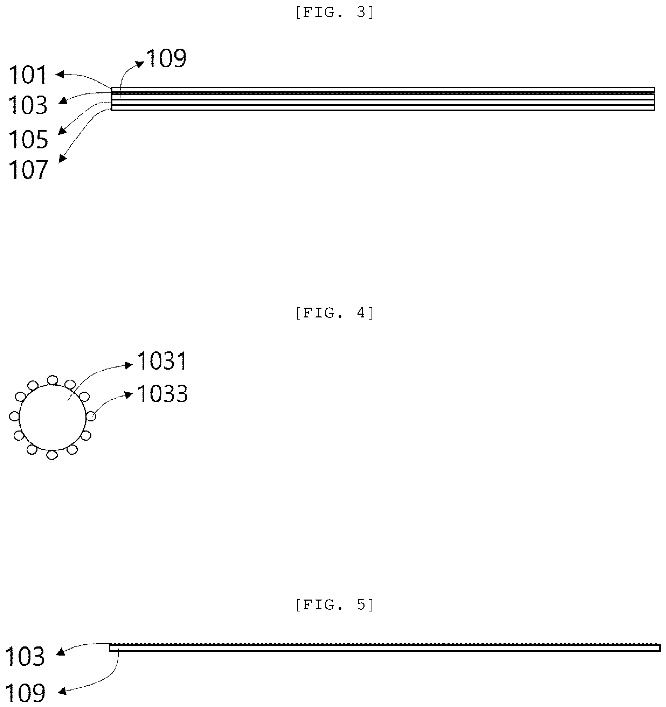

Hereinafter, the configuration of the mask body 100 according to another exemplary embodiment the present disclosure will be described in more detail with reference to FIG. 3. FIG. 3 is a view illustrating another exemplary embodiment of a cross section of the mask body taken along line S-S' in FIG. 1.

Referring to FIG. 3, the mask body 100 according to another exemplary embodiment may include an inner fabric 107; a filter 105 formed on the inner fabric 107; an intermediate material 109 formed on the filter 105; and an outer fabric 101 formed on the intermediate material 109, and the antibacterial deodorizing layer 103 may be formed on the intermediate material 109 so as to be directed toward the outer fabric 101.

The inner fabric 107, the filter 105, and the outer fabric 101 may be substantially the same as those of the antibacterial deodorizing mask including titanium dioxide according to the above-mentioned exemplary embodiment.

The intermediate material 109 may adopt spun bond as a non-woven fabric, and for example, spun bond of 20 g to 40 g may be applied.

The antibacterial deodorizing layer 103 may be formed on the intermediate material 109 so as to be directed toward the outer fabric 101. In this case, the intermediate material 109 and the antibacterial deodorizing layer 103 may be manufactured in advance as a separate antibacterial deodorizing pad and then used for the process of manufacturing the mask. The method of fixing and forming the titanium dioxide particles of the antibacterial deodorizing layer 103 on the intermediate material 109 is substantially the same as that of the antibacterial deodorizing mask including titanium dioxide according to the above-mentioned exemplary embodiment. The method of separately manufacturing the antibacterial deodorizing pad and then manufacturing the mask may further improve power of blocking viruses, fine dust, yellow dust, and respiratory droplets and may more effectively prevent the wearer from inhaling the titanium dioxide particles.

Even in this case, the filter 105 and the outer fabric 101 may also be coupled by ultrasonic bonding 140, and specific details are substantially the same as those of the antibacterial deodorizing mask including titanium dioxide according to the above-mentioned exemplary embodiment.

Hereinafter, the titanium dioxide particles according to another exemplary embodiment of the present disclosure will be described with reference to FIG. 4. FIG. 4 is a view simply illustrating a titanium dioxide particle according to another exemplary embodiment of the present disclosure; and

According to the titanium dioxide particle according to another exemplary embodiment, loess, charcoal, activated carbon, terra alba, zeolite, and/or lime particles 1033 may be attached to a surface of a titanium dioxide particle 1031 having an average particle diameter D50 of 0.2 mm to 2 mm. The loess, the charcoal, the activated carbon, the terra alba, the zeolite, and/or the lime particles 1033 may further improve the antibacterial and deodorizing effect and prevent the titanium dioxide particles from being separated from the mask.

In this case, a ratio of the average particle diameter D50 of the loess, the charcoal, the activated carbon, the terra alba, the zeolite, and the lime particles 1033 to the average particle diameter D50 of the titanium dioxide may be 0.1:1 to 0.3:1. In this range of the ratio of the average particle diameter, the titanium dioxide particles are less separated because of unevenness of the surface of the particle, and it is possible to basically prevent the wearer from inhaling the titanium dioxide particles.

The loess, the charcoal, the activated carbon, the terra alba, the zeolite, and/or the lime particles 1033 may be attached to the titanium dioxide 1031 by means of plastic clay, and for example, the plastic clay may include at least one of bentonite and montmorillonite. The plastic clay may fix the loess, the charcoal, the activated carbon, the terra alba, the zeolite, and the lime particles to the titanium dioxide only by a drying process without performing a firing process, and the plastic clay is a substance harmless to the human body.

### TITANIUM DIOXIDE PARTICLES AND METHOD OF MANUFACTURING SAME

The titanium dioxide particles having the average particle diameter D50 of 0.2 mm to 2 mm may be made of a composition for forming titanium dioxide, and the composition for forming the titanium dioxide may include 85 to 95% by weight of titanium dioxide particles having an average particle diameter D50 of 1 µm or less and 5 to 15% by weight of magnesium. The magnesium content may affect the particle diameter of the titanium dioxide particles, and particularly, in the above-mentioned content range, the titanium dioxide and the magnesium may become at least partially eutectic, thereby reducing a process temperature and manufacturing the titanium dioxide particles having a predetermined size or larger. In addition, the magnesium has an effect of improving catalytic efficiency in a visible light region and catalytic efficiency at a lower electric current, thereby further improving the antibacterial deodorizing effect.

The titanium dioxide particles may be manufactured by a method including: mixing 85 to 95% by weight of titanium dioxide particles having an average particle diameter D50 of 1 µm or less and 5 to 15% by weight of magnesium; heating the mixture in a tube furnace under a H₂/Ar atmosphere at 500°C to 550°C for 4 to 6 hours; stirring the mixture in a 1.0 M hydrogen chloride (HCl) solution for 11 to 13 hours; removing acid by washing the mixture with water; and drying the mixture.

### ANTIBACTERIAL DEODORIZING PAD

Another aspect of the present disclosure relates to an antibacterial deodorizing pad. Referring to FIG. 5, the antibacterial deodorizing pad may include an intermediate material layer 109 including spun bond, and an antibacterial deodorizing layer 103 provided on the intermediate material layer 109 and including titanium dioxide particles having an average particle diameter D50 of 0.2 mm to 2 mm.

The configuration in which the antibacterial deodorizing layer 103 is formed on the intermediate material layer 109 is substantially the same as the above-mentioned configuration of the antibacterial deodorizing mask including titanium dioxide.

The antibacterial deodorizing pad is formed by forming the antibacterial deodorizing layer 103 on the intermediate material layer 109 first, such that the antibacterial deodorizing pad has an advantage of further simplifying the process of manufacturing the mask.

While FIG. 5 illustrates the example in which the antibacterial deodorizing layer 103 is formed on the intermediate material layer 109, an antibacterial deodorizing pad having the antibacterial deodorizing layer 103 formed on the outer fabric 101 and the filter 105 may be applied.

While the exemplary embodiments of the present disclosure have been described above, the present disclosure is not limited to the exemplary embodiments disclosed above but will be implemented in various different forms, and those skilled in the art will understand that the present disclosure may be implemented in any other specific form without changing the technical spirit or the essential feature of the present disclosure. Therefore, it should be understood that the above-described exemplary embodiments are illustrative in all aspects and do not limit the present application.

## Claims

1. An antibacterial deodorizing mask including titanium dioxide, the antibacterial deodorizing mask comprising:
a mask body configured to surround at least one of a wearer's mouth and nose; and
a pair of ear loops provided at both sides of the mask body so as to be hung on the wearer's ears and each having both ends coupled to upper and lower portions of the mask body, respectively,
wherein the mask body comprises an antibacterial deodorizing layer comprising titanium dioxide particles having an average particle diameter D50 of 0.2 mm to 2 mm.

2. The antibacterial deodorizing mask of claim 1, wherein the mask body comprises: an inner fabric; a filter formed on the inner fabric; and an outer fabric formed on the filter, and
wherein the antibacterial deodorizing layer is formed on the filter so as to be directed toward the outer fabric or formed on the outer fabric so as to be directed toward the filter.

3. The antibacterial deodorizing mask of claim 2, wherein the filter and the outer fabric are at least partially coupled by ultrasonic bonding.

4. The antibacterial deodorizing mask of claim 1, wherein the mask body comprises: an inner fabric; a filter formed on the inner fabric; an intermediate material formed on the filter; and an outer fabric formed on the intermediate material, and
wherein the antibacterial deodorizing layer is formed on the intermediate material so as to be directed toward the outer fabric.

5. The antibacterial deodorizing mask of claim 4, wherein the intermediate material and the outer fabric are at least partially coupled by ultrasonic bonding.

6. The antibacterial deodorizing mask of any one of claim 1 to 5, wherein loess, charcoal, activated carbon, terra alba, zeolite, and lime particles are attached to surfaces of the titanium dioxide particles by means of at least one of bentonite and montmorillonite.
